# EUROPEAN PATENT SPECIFICATION

(11) **EP 1 674 057 B1**
(45) Date of publication and mention of the grant of the patent: **20.08.2008**
(21) Application number: 04030643.3
(22) Date of filing: 23.12.2004
(51) Int. Cl.: A61F 11/06

(54) **Hearing protection device and use of such a device**
Gehörschutzvorrichtung und Verwendung dieser Vorrichtung
Dispositif de protection auditive et l'utilisation d'un tel dispositif

(43) Date of publication of application: 28.06.2006
(73) Proprietor: PHONAK AG, 8712 Stäfa (CH)
(72) Inventor: Berg, Christian, 8713 Uerikon (CH)
(74) Representative: Schwan - Schwan - Schorer

(56) References cited:
- WO-A-02/17839
- WO-A-94/05231
- DE-A1- 19 926 820
- GB-A- 2 373 951
- US-A1- 2002 080 979
- US-A1- 2003 059 071

## Description

The present invention relates to a hearing protection device according to the preamble of claim 1.

US 5,426,719 relates to a hearing protection device comprising two customized earplugs which both are connected via a respective wiring to an electronic module worn below the user's chin. One of the earplugs includes a speaker while the other earplug includes a microphone. The electronic module contains electronic components such as a power supply, a microphone preamplifier, equalization circuitry and radio receiver and transmitter. An antenna for radio frequency reception and transmission of speech is included in the wiring connecting the earplugs to the electronic module. The system is designed for allowing acoustic communication between several workers wearing the earplugs by radio frequency communication via the antenna included in the wiring.

DE 101 17 705 A1 relates to a hearing protection device including a combination of earmuffs and earplugs. The earplugs include a speaker, while the earmuffs include a microphone and an electronic unit for providing an active hearing protection function. The microphone and the speaker are coupled by coils. In addition, the electronic unit may be connected to a radio frequency antenna which may be integrated in the headband connecting the earmuffs in order to connect the electronic unit to a radio frequency transmitter for external communication purposes, such as connection to a phone system.

WO 01/66054 A1 relates to a wireless headset wherein the headband of the earmuffs includes an antenna.

GB 2 373 951 A relates to hearing protection device according to the preamble of claim 1, wherein each of the two earplugs comprises a built-in communication system which comprises an internal speaker and microphone that are either hard wired to a radio system or have a wireless configuration.

US 2002/0080979 A1 relates to a hearing protection system comprising two earplugs, with each earplug comprising an active unit having a microphone and a loudspeaker for electronically transmitting ambient sound through the earplug. The active units are connected by a flexible mechanical connection into which wires for connecting the active units to electronic components, such as a battery, shared by the active units are integrated.

It is an object of the invention to provide for a hearing protection device comprising two earplugs which, in addition to the hearing protection function, provides for a communication function but nevertheless has a compact and simple mechanical construction. It is another object of the invention to provide for a corresponding use of such hearing protection devices.

According to the invention, these objects are achieved by a hearing protection device as defined in claims 1 and a use of such hearing protection device as defined in claims 25and 27, respectively.

The invention in general is beneficial in that by integrating an antenna into the flexible means for mechanically connecting the two earplugs, the earplugs are provided with an added communication function without the need for additional mechanical parts. The invention is further beneficial in that, by integrating an electromagnetic signal receiving unit or electromagnetic signal transmitting unit necessary for communication via the antenna, into at least one of the earplugs, the complete minimum arrangement necessary for the desired communication is provided within the hearing protection device without additional external elements being necessary. Thereby the invention provides for particularly compact hearing protection devices having an added communication function.

The invention is particularly useful if speech or other sound signal perception by the user is desired in a noisy environment which requires the use of a hearing protection device or if the user, in a noisy environment which requires the use of hearing protection devices, wants to provide a remote person with a speech signal.

Preferred embodiments of the invention are defined in the dependent claims.

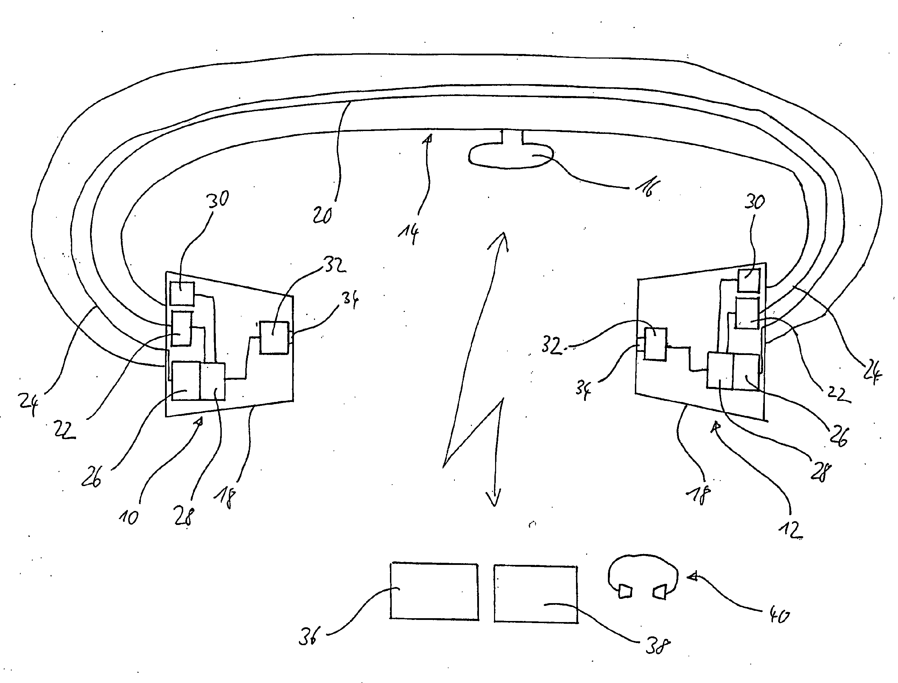

In the following, examples of the invention will be illustrated by reference to the attached single drawing which shows a schematic view of an example of a hearing protection device according to the invention.

The invention generally relates to hearing protection devices comprising two hearing protection earplugs which are to be worn at least in part in the user's right and left ear canal, respectively, i.e. at least the distal part of one of the earplugs is to be worn in the user's right ear canal while the distal part of the other one of the earplugs is to be worn in the left ear canal. The two earplugs are mechanically connected by flexible means, for example a cord or a headband. Such mechanical connecting means are provided for keeping the earplugs together and to prevent them from being lost. Preferably the mechanical connection means comprises means for fastening the mechanical connection means, and hence the earplugs, at the user's clothing. This is particularly important in view of the fact that hearing protection devices usually are used in rough environments and/or in situations of stress and distraction of the user so that hearing protection devices are particularly susceptible to being lost. Preventing loss of hearing protection devices is of crucial importance in the food processing industry, where contamination of the processed materials by lost articles is to be strictly avoided.

According to the single figure, a right ear hearing protection earplug 10 is mechanically connected to a left ear hearing protection earplug 12 by a flexible mechanical connection element 14, such as a headband or a cord. The connection element 14 is provided with a fixing element 16 which serves for fastening the connection 14 to the user's clothing. The fixing element 16 may be designed, for example, as a clip.

Each earplug 10, 12 comprises a shell 18 having an outer surface which is shaped such that the earplug 10, 12 can be inserted into the user's ear canal in order to provide for a hearing protection function by providing for an acoustic attenuation of preferably at least 10 dB averaged over the audible frequency range when the earplug is worn by the user to protect the user from excessive levels of ambient sound.

The earplug may comprise an acoustic filter for adjusting the desired total acoustic attenuation or for adjusting the frequency dependent acoustic attenuation.

The shell preferably is a hard shell having an elasticity from shore D85 to D65 and preferably is made of polyamide. In order to achieve optimized fit of the shell within the user's outer ear and ear canal, the shell preferably has an outer surface individually shaped according to the measured shape of the user's outer ear and ear canal, i.e. the shell preferably has an individually customized outer shape. The shape of the user's outer ear and ear canal may be determined by direct three-dimensional scanning of the ear canal and the concha or by producing an impression of the ear canal and the concha which subsequently undergoes scanning. The scanning process may be carried out optically, preferably by laser scanning. The digital data obtained by the scanning process is then used to create the hard shell by an additive or incremental layer-by-layer build up process. Such processes are also known as "rapid prototyping".

A preferred additive build-up process is a layer-by-layer laser sintering process of powder material, preferably polyamide powder. Such processes are also known as "selective laser sintering" (SLS). The basic principle therein is the repeated deposition of a thin layer of material on a surface, with the desired sectional shape then being stabilized, i.e. hardened, by laser action. An overview regarding such processes can be found, for example, in US 2003/0133583 A1 or US 6,533,062 B1.

In order to allow the user to communicate while wearing the earplugs, the hearing protection device of the present invention is provided with active components. For example, one or both of the earplugs may be provided with a microphone for converting ambient sound into input audio signals, a digital processing unit for processing these input audio signals into output audio signals, and an acoustic output transducer for converting the output audio signals into sound to be perceived by the user when wearing the earplugs. Such a configuration is also known as an active hearing protection device which enables the user to perceive ambient sound, for example speech signals, in a controlled manner while wearing the hearing protection device. Usually, the total amplification provided by the microphone, the digital signal processing unit and the output transducer is negative.

The connection 14 may be provided with a binaural communication link 20 for enabling the electronic units of the two earplugs 10, 12 to exchange data via the communication link 20 in order to provide for a binaural hearing function. To this end, both earplugs 10, 12 are provided with an electronic communication unit 22 which is connected with the communication link 20 in order to exchange data between the two earplugs 10, 12. Preferably, the communication link 20 is an optical fibre connection or a wire connection integrated within the mechanical connection 14.

According to one aspect of the invention, one or both of the earplugs 10, 12 is/are provided with an electromagnetic signal receiving unit and/or an electromagnetic signal transmitting unit in order to allow for wireless electromagnetic communication of the hearing protection device with a remote unit via an antenna 24 integrated within the mechanical connection 14. Preferably the antenna, the receiving unit and/or the transmitting unit are adapted to operate at radio frequencies. The antenna may be designed as an inductive loop antenna.

According to a preferred embodiment, the hearing protection device is adapted for bidirectional wireless communication with the remote unit via the antenna. In this case, at least one of the earplugs will be equipped with a microphone in order to transmit speech signals produced by the user of the hearing protection device to the remote unit, which preferably is, in this case, an active hearing protection device worn by another user, so that the two users are able to communicate by speech with each other via the bidirectional wireless communication link. In this case, electromagnetic signals corresponding to speech signals sensed by the microphone are transmitted to the other user via the antenna of the hearing protection device, while speech signals sent from the hearing protection device of the other user are received via the antenna and can be perceived by the user via the speakers within the earplugs.

However, in some cases it might be desirable to equip the hearing protection device only for unidirectional wireless communication, for example, by providing a hearing protection system with speakers only or with microphones only.

The invention is not restricted to the case in which the remote unit is another hearing protection device. Rather, the remote unit, both in case of bidirectional or unidirectional communication, may be for example an audio signal source such as a mobile phone, a personal computer, a discman, an MP3 player, a CD player, a DVD player or a radio tuner or an audio signal source permanently installed in a building.

However, the wireless communication link via the antenna may be used not only for transmitting or receiving audio signals but also might be used for exchange of non-audio data. In this case, the remote unit may be used, for example, for programming or configuring a digital signal processing unit provided within at least one of the earplugs.

In the single figure an example is shown in which both earplugs 10, 12 are adapted for full bidirectional wireless communication with a remote unit and where in addition the two earplugs 10, 12 are connected by the binaural communication link 20 for achieving binaural functionality of the overall system.

According to the figure, each earplug 10, 12 is provided with a unit 22 for bidirectional electronic communication via the communication link 20, an electromagnetic signal receiving and transmitting unit 26 including a digital signal processing unit 28, a microphone 30 for converting ambient sound into input audio signals for the digital signal processing unit 28, and an output transducer (speaker) 32 which is acoustically connected to a sound outlet opening 34 provided at the distal end of the earplug 10, 12. The speaker 32 is provided with output audio signals from the digital signal processing unit 28 which receives input audio signals both from the microphone 30 and the receiving/transmitting unit 26. The receiving/transmitting unit 26 of each earplug is connected to the antenna 24 which may be common to both receiving/transmitting units 26.

Further, the figure schematically shows examples of the remote unit, such as a remote audio signal source 36 which may be a mobile device or which may be permanently installed in a building, a remote programming/configuring source 38 or a remote active hearing protection device 40 worn by another user.

A power source for the electronic components may be included within the earplugs 10, 12 or it may be provided externally, such as within the fixing element 16, wherein in the latter case corresponding power supply wires would be integrated within the mechanical connection 14 (not shown in the figure).

Optionally, the antenna 24 and the communication link 20 could be physically the same element, i.e. the antenna could also serve as the communication link between the earplugs 10, 12.

## Claims

1. Hearing protection device comprising two earplugs (10, 12) to be worn at least in part in a user's right and left ear canal, respectively, wherein at least one of said earplugs comprises an acoustic output transducer (32) and/or a microphone (30), , wherein at least one of said earplugs comprises an electromagnetic signal receiving unit (26) electrically connected to said output transducer and/or an electromagnetic signal transmitting unit (26) electrically connected to said microphone, **characterized by** flexible means (14) for mechanically connecting the two earplugs, said connecting means comprising an antenna (24), said electromagnetic signal receiving unit and said electromagnetic signal transmitting unit, respectively, being electrically connected to said antenna, and said hearing protection device being adapted for wireless electromagnetic communication with a remote unit (36, 38, 40) via said antenna.

2. Hearing protection device of claim 1, wherein said antenna (24) and said receiving unit (26) are adapted to operate at radio frequencies.

3. Hearing protection device of claim 1, wherein said antenna (24) is designed as an inductive loop antenna.

4. Hearing protection device of one of the preceding claims, wherein said receiving unit (26) comprises a digital signal processing unit (28).

5. Hearing protection device of one of the preceding claims, wherein both earplugs (10, 12) comprise an acoustic output transducer (32).

6. Hearing protection device of one of the preceding claims, wherein both earplugs (10, 12) comprise an electromagnetic signal receiving unit (26).

7. Hearing protection device of one of the preceding claims, wherein both earplugs (10, 12) comprise a digital signal processing unit (28).

8. Hearing protection device of claim 7, wherein both earplugs (10, 12) comprise a microphone (30) for producing audio signals, said digital signal processing unit (28) being adapted to process said audio signals as input to said output transducer (32).

9. Hearing protection device of claim 8, wherein the total amplification provided by said microphone (30), said digital signal processing unit (28) and said output transducer (32) is negative.

10. Hearing protection device of one of the preceding claims, wherein said hearing protection device is adapted for bidirectional wireless electromagnetic communication with said remote unit (36, 38, 40) via said antenna (24).

11. Hearing protection device of one of the preceding claims, wherein at least one of said earplugs comprises an electromagnetic signal transmitting unit.

12. Hearing protection device of claim 11, wherein said electromagnetic signal transmitting unit (26) is adapted to operate at radio frequencies.

13. Hearing protection device of one of the preceding claims, wherein said remote unit is an active hearing protection device (40) worn by another user.

14. Hearing protection device of one of claims 1 to 12, wherein said remote unit is an audio signal source (36) such as a mobile phone, a personal computer, a discman, an MP3 player, a CD player, a DVD player or a radio tuner or an audio signal source permanently installed in a building.

15. Hearing protection device of one of claims 1 to 12, wherein said remote unit is a unit (38) for programming and/or configuring said digital signal processing unit (28).

16. Hearing protection device of one of the preceding claims, wherein both earplugs (10, 12) comprise means (22) for electronic communication and said mechanical connecting means (14) comprises a binaural communication link (20) for directly connecting said communication means of said two earplugs.

17. Hearing protection device of one of the preceding claims, wherein both earplugs (10, 12) comprise an acoustic output transducer (32) and means (22) for electronic communication, and wherein said mechanical connecting means (14) comprises a binaural communication link (20) for directly connecting said communication means of said two earplugs.

18. Hearing protection device of claim 17, wherein said binaural communication link (20) is an optical fiber connection or a wire connection integrated within said mechanical connection means.

19. Hearing protection device of one of claims 17 or 18, wherein each of said means (22) for electronic communication comprises a digital signal processing unit.

20. Hearing protection device of one of the preceding claims, wherein said mechanical connection means (14) is a headband or a cord.

21. Hearing protection device of one of the preceding claims, wherein said mechanical connection means (14) comprises means (16) for fastening said mechanical connection means to the user's clothing.

22. Hearing protection device of one of the preceding claims, wherein each of said earplugs (10, 12) comprises a shell (18) which is designed for achieving an acoustic attenuation of at least 10 dB averaged over the audible frequency range when worn by the user.

23. Hearing protection device of one of the preceding claims, wherein the outer surface of said shell (18) is individually shaped according to the measured inner shape of the user's outer ear and ear canal.

24. Hearing protection device of claim 23, wherein said shell (18) is a hard shell with an elasticity of from shore D85 to shore D65.

25. Use of a hearing protection device of one of claims 1 to 24, comprising: inserting said earplugs (10, 12) into said user's ear canal for mechanically attenuating sound waves arriving at the outer end of each earplug by at least 10 dB averaged over the audible frequency range, establishing a wireless electromagnetic communication between said hearing protection device and said remote unit (36, 38, 40) via said antenna (24), and providing acoustic signals to the user's ear via said at least one acoustic output transducer (32).

26. Use of claim 25, further comprising: programming and/or configuring a digital signal processing unit (28) provided in at least one of said earplugs (10, 12) by signals from said remote unit (38) via said antenna (24).

27. Use of a hearing protection device of one of claims 1 to 24, comprising: inserting said earplugs (10, 12) into said user's ear canal for mechanically attenuating sound waves arriving at the outer end of each earplug by at least 10 dB averaged over the audible frequency range, establishing a wireless electromagnetic communication between said hearing protection device and said remote unit (40) via said antenna (24), transmitting audio signals generated by said microphone (30) to said remote unit , and providing said audio signals to the ear of another user via at least one acoustic output transducer.

## Patentansprüche

1. Gehörschutz mit zwei Ohrstöpseln (10, 12), die mindestens teilweise im rechten bzw. linken Gehörgang des Anwenders zu tragen sind, wobei mindestens einer der Ohrstöpsel einen akustischen Ausgangswandler (32) und/oder ein Mikrophon (30) aufweist, und wobei mindestens einer der Ohrstöpsel eine Einheit (26) zum Empfangen eines elektromagnetischen Signals, die mit dem Ausgangswandler elektrisch verbunden ist, und/oder eine Einheit (26) zum Senden eines elektromagnetischen Signals, welche elektrisch mit dem Mikrophon verbunden ist, aufweist, **gekennzeichnet durch** eine flexible Anordnung (14) zum mechanischen Verbinden der beiden Ohrstöpsel, die eine Antenne (24) aufweist, wobei die Einheit zum Empfangen eines elektromagnetischen Signals bzw. die Einheit zum Senden eines elektromagnetischen Signals mit der Antenne elektrisch verbunden sind, und wobei der Gehörschutz für drahtlose elektromagnetische Kommunikation mit einer externen Einheit (36, 38, 40) über die Antenne ausgebildet ist.

2. Gehörschutz gemäß Anspruch 1, wobei die Antenne (24) und die Empfangseinheit (26) für den Betrieb bei Radiofrequenzen ausgebildet sind.

3. Gehörschutz gemäß Anspruch 1, wobei die Antenne (24) als eine Induktionsschleifenantenne ausgebildet ist.

4. Gehörschutz gemäß einem der vorhergehenden Ansprüche, wobei die Empfangseinheit (26) eine digitale Signalverarbeitungseinheit (28) aufweist.

5. Gehörschutz gemäß einem der vorhergehenden Ansprüche, wobei beide Ohrstöpsel (10, 12) einen akustischen Ausgangswandler (32) aufweisen.

6. Gehörschutz gemäß einem der vorhergehenden Ansprüche, wobei beide Ohrstöpsel (10, 12) eine Einheit (26) zum Empfangen eines elektromagnetischen Signals aufweisen.

7. Gehörschutz gemäß einem der vorhergehenden Ansprüche, wobei beide Ohrstöpsel (10, 12) eine digitale Signalverarbeitungseinheit (28) aufweisen.

8. Gehörschutz gemäß Anspruch 7, wobei beide Ohrstöpsel (10, 12) ein Mikrofon (30) zum Erzeugen von Audiosignalen aufweisen und wobei die digitale Signalverarbeitungseinheit (28) ausgebildet ist, um die Audiosignale als Eingangssignal für den Ausgangswandler (32) zu verarbeiten.

9. Gehörschutz gemäß Anspruch 8, wobei die mittels des Mikrofons (30), der digitalen Signalverarbeitungseinheit (28) und dem Ausgangswandler (32) erzielte Gesamtverstärkung negativ ist.

10. Gehörschutz gemäß einem der vorhergehenden Ansprüche, wobei der Gehörschutz für bidirektionale drahtlose elektromagnetische Kommunikation mit der externen Einheit (36, 38, 40) über die Antenne (24) ausgebildet ist.

11. Gehörschutz gemäß einem der vorhergehenden Ansprüche, wobei mindestens einer der Ohrstöpsel eine Einheit zum Senden eines elektromagnetischen Signals aufweist.

12. Gehörschutz gemäß Anspruch 11, wobei die Einheit (26) zum Senden eines elektromagnetischen Signals für den Betrieb bei Radiofrequenzen ausgebildet ist.

13. Gehörschutz gemäß einem der vorhergehenden Ansprüche, wobei die externe Einheit als aktiver Gehörschutz (40) ausgebildet ist, der von einem anderen Anwender getragen wird.

14. Gehörschutz gemäß einem der Ansprüche 1 bis 12, wobei es sich bei der externen Einheit um eine Audiosignalquelle (36), wie beispielsweise ein Mobiltelefon, einen PC, einen Disc-Man, einen MP3-Spieler, einen CD-Spieler, einen DVD-Spieler oder einen Radioempfänger oder um eine dauerhaft in einem Gebäude installierte Audiosignalquelle handelt.

15. Gehörschutz gemäß einem der Ansprüche 1 bis 12, wobei es sich bei der externen Einheit um eine Einheit (38) zum Programmieren und/oder Konfigurieren der digitalen Signalverarbeitungseinheit (28) handelt.

16. Gehörschutz gemäß einem der vorhergehenden Ansprüche, wobei beide Ohrstöpsel (10, 12) Mittel (22) zur elektronischen Kommunikation aufweisen und wobei die mechanische Verbindungsanordnung (14) eine binaurale Kommunikationsstrecke (20) zum direkten Verbinden der Kommunikationsmittel der beiden Ohrstöpsel aufweist.

17. Gehörschutz gemäß einem der vorhergehenden Ansprüche, wobei beide Ohrstöpsel (10, 12) einen akustischen Ausgangswandler (32) und Mittel (22) zur elektronischen Kommunikation aufweisen und wobei die mechanische Verbindungsanordnung (14) eine binaurale Kommunikationstrecke (20) zum direkten Verbinden der Kommunikationsmittel der beiden Ohrstöpsel aufweist.

18. Gehörschutz gemäß Anspruch 17, wobei es sich bei der binauralen Kommunikationsstrecke (20) um eine in die mechanische Verbindungsanordnung integrierte Lichtleiterverbindung oder Drahtverbindung handelt.

19. Gehörschutz gemäß einem der Ansprüche 17 oder 18, wobei jedes der Mittel (22) zur elektronischen Kommunikation eine digitale Signalverarbeitungseinheit aufweist.

20. Gehörschutz gemäß einem der vorhergehenden Ansprüche, wobei es sich bei der mechanischen Verbindungsanordnung (14) um ein Kopfband oder eine Schnur handelt.

21. Gehörschutz gemäß einem der vorhergehenden Ansprüche, wobei die mechanische Verbindungsanordnung (14) Mittel (16) zum Befestigen der mechanischen Verbindungsanordnung an der Kleidung des Anwenders aufweist.

22. Gehörschutz gemäß einem der vorhergehenden Ansprüche, wobei jeder der Ohrstöpsel (10, 12) eine Schale (18) aufweist, die ausgebildet ist, um eine akustische Dämpfung von mindestens 10 dB, gemittelt über den hörbaren Frequenzbereich, zu erzielen, wenn sie vom Anwender getragen wird.

23. Gehörschutz gemäß einem der vorhergehenden Ansprüche, wobei die Außenfläche der Schale (18) gemäß der gemessenen inneren Form des Außenohrs und Gehörgangs des Anwenders individuell geformt ist.

24. Gehörschutz gemäß Anspruch 23, wobei es sich bei der Schale (18) um eine harte Schale mit einer Elastizität im Bereich von Shore D85 bis Shore D65 handelt.

25. Verwendung eines Gehörschutzes gemäß einem der Ansprüche 1 bis 24, wobei die Ohrstöpsel (10, 12) in den Gehörgang des Anwenders eingeführt werden, um am äußeren Ende eines jeden Ohrstöpsels auftreffende Schallwellen um mindestens 10 dB, gemittelt über den hörbaren Frequenzbereich, mechanisch zu dämpfen, wobei drahtlose elektromagnetische Kommunikation zwischen dem Gehörschutz und der externen Einheit (36, 38, 40) über die Antenne (24) aufgebaut wird, und wobei dem Ohr des Anwenders über den mindestens einen akustischen Ausgangswandler (32) akustische Signale zugeführt werden.

26. Verwendung gemäß Anspruch 25, wobei ferner eine digitale Signalverarbeitungseinheit (28), die in mindestens einem der Ohrstöpsel (10, 12) vorgesehen ist, mittels Signalen von der externen Einheit (38) über die Antenne (24) programmiert und/oder konfiguriert wird.

27. Verwendung eines Gehörschutzes gemäß einem der Ansprüche 1 bis 24, wobei die Ohrstöpsel (10, 12) in den Gehörgang des Anwenders eingeführt werden, um am äußeren Ende eines jeden Ohrstöpsels auftreffende Schallwellen um mindestens 10 dB, gemittelt über den hörbaren Frequenzbereich, mechanisch zu dämpfen, wobei drahtlose elektromagnetische Kommunikation zwischen dem Gehörschutz und der externen Einheit (40) über die Antenne (24) aufgebaut wird, wobei von dem Mikrofon (30) erzeugte Audiosignale an die externe Einheit gesendet werden, und wobei die Audiosignale dem Ohr eines anderen Anwenders über mindestens einen akustischen Ausgangswandler zugeführt werden.

## Revendications

1. Dispositif de protection de l'ouïe comprenant deux bouchons d'oreilles (10, 12) destinés à être portés au moins en partie et respectivement dans le canal intra-auriculaire gauche et droit d'un utilisateur, dans lequel au moins l'un desdits bouchons d'oreilles comprend un transducteur de sortie acoustique (32) et/ou un microphone (30), dans lequel au moins l'un desdits bouchons d'oreilles comprend une unité de réception de signaux électromagnétiques (26) reliée électriquement audit transducteur de sortie et/ou une unité de transmission de signaux électromagnétiques (26) reliée électriquement audit microphone, **caractérisé par** un moyen flexible (14) pour relier mécaniquement les deux bouchons d'oreilles, ledit moyen de connexion comprenant une antenne (24), ladite unité de réception de signaux électromagnétiques et ladite unité de transmission de signaux électromagnétiques, respectivement, étant reliées électriquement à ladite antenne, et ledit dispositif de protection de l'ouïe étant apte à une communication électromagnétique sans fil avec une unité distante (36, 38, 40) via ladite antenne.

2. Dispositif de protection de l'ouïe selon la revendication 1, dans lequel ladite antenne (24) et ladite unité de réception (26) sont aptes à fonctionner à des radiofréquences.

3. Dispositif de protection de l'ouïe selon la revendication 1, dans lequel ladite antenne (24) est conçue comme une antenne à boucle d'induction.

4. Dispositif de protection de l'ouïe selon l'une quelconque des revendications précédentes, dans lequel ladite unité de réception (26) comprend une unité de traitement de signaux numériques (28).

5. Dispositif de protection de l'ouïe selon l'une quelconque des revendications précédentes, dans lequel les deux bouchons d'oreilles (10, 12) comprennent un transducteur de sortie acoustique (32).

6. Dispositif de protection de l'ouïe selon l'une quelconque des revendications précédentes, dans lequel les deux bouchons d'oreilles (10, 12) comprennent une unité de réception de signaux électromagnétiques (26).

7. Dispositif de protection de l'ouïe selon l'une quelconque des revendications précédentes, dans lequel les deux bouchons d'oreilles (10, 12) comprennent une unité de traitement de signaux numériques (28).

8. Dispositif de protection de l'ouïe selon la revendication 7, dans lequel les deux bouchons d'oreilles (10, 12) comprennent un microphone (30) pour produire des signaux audio, ladite unité de traitement de signaux numériques (28) étant apte à traiter lesdits signaux audio en tant qu'entrée dans ledit transducteur de sortie (32).

9. Dispositif de protection de l'ouïe selon la revendication 8, dans lequel l'amplification totale fournie par ledit microphone (30), ladite unité de traitement de signaux numériques (28) et ledit transducteur de sortie (32) est négative.

10. Dispositif de protection de l'ouïe selon l'une quelconque des revendications précédentes, dans lequel ledit dispositif de protection de l'ouïe est apte à une communication électromagnétique sans fil bidirectionnelle avec ladite unité distante (36, 38, 40) via ladite antenne (24).

11. Dispositif de protection de l'ouïe selon l'une quelconque des revendications précédentes, dans lequel au moins l'un desdits bouchons d'oreilles comprend une unité de transmission de signaux électromagnétiques.

12. Dispositif de protection de l'ouïe selon la revendication 11, dans lequel ladite unité de transmission de signaux électromagnétiques (26) est apte à fonctionner à des radiofréquences.

13. Dispositif de protection de l'ouïe selon l'une quelconque des revendications précédentes, dans lequel ladite unité distante est un dispositif de protection de l'ouïe actif (40) porté par un autre utilisateur.

14. Dispositif de protection de l'ouïe selon l'une des revendications 1 à 12, dans lequel ladite unité distante est une source de signaux audio (36) telle qu'un téléphone mobile, un ordinateur personnel, un baladeur CD, un baladeur MP3, un lecteur de CD, un lecteur de DVD ou un tuner radio ou une source de signaux audio installée à demeure dans un bâtiment.

15. Dispositif de protection de l'ouïe selon l'une des revendications 1 à 12, dans lequel ladite unité distante est une unité (38) pour programmer et/ou configurer ladite unité de traitement de signaux numériques (28).

16. Dispositif de protection de l'ouïe selon l'une quelconque des revendications précédentes, dans lequel les deux bouchons d'oreilles (10, 12) comprennent des moyens (22) pour la communication électronique et ledit moyen de connexion mécanique (14) comprend une liaison de communication binaurale (20) pour relier directement ledit moyen de communication desdits deux bouchons d'oreilles.

17. Dispositif de protection de l'ouïe selon l'une quelconque des revendications précédentes, dans lequel les deux bouchons d'oreilles (10, 12) comprennent un transducteur de sortie acoustique (32) et des moyens (22) pour la communication électronique, et dans lequel ledit moyen de connexion mécanique (14) comprend une liaison de communication binaurale (20) pour relier directement ledit moyen de communication desdits deux bouchons d'oreilles.

18. Dispositif de protection de l'ouïe selon la revendication 17, dans lequel ladite liaison de communication binaurale (20) est une connexion par fibre optique ou une connexion par fil intégrée au sein dudit moyen de connexion mécanique.

19. Dispositif de protection de l'ouïe selon la revendication 17 ou 18, dans lequel chacun desdits moyens (22) pour la communication électronique comprend une unité de traitement de signaux numériques.

20. Dispositif de protection de l'ouïe selon l'une quelconque des revendications précédentes, dans lequel ledit moyen de connexion mécanique (14) est un serre-tête ou un cordon.

21. Dispositif de protection de l'ouïe selon l'une quelconque des revendications précédentes, dans lequel ledit moyen de connexion mécanique (14) comprend un moyen (16) pour fixer ledit moyen de connexion mécanique aux vêtements de l'utilisateur.

22. Dispositif de protection de l'ouïe selon l'une quelconque des revendications précédentes, dans lequel chacun desdits bouchons d'oreilles (10, 12) comprend une coque (18) qui est conçue pour obtenir une atténuation acoustique d'au moins 10 dB en moyenne dans la gamme des fréquences audibles lorsque portée par l'utilisateur.

23. Dispositif de protection de l'ouïe selon l'une quelconque des revendications précédentes, dans lequel la surface externe de ladite coque (18) est façonnée individuellement en fonction de la forme interne mesurée du canal externe et interne de l'oreille de l'utilisateur.

24. Dispositif de protection de l'ouïe selon la revendication 23, dans lequel ladite coque (18) est une coque dure ayant une élasticité comprise entre Shore D85 et Shore D65.

25. Utilisation d'un dispositif de protection de l'ouïe selon l'une des revendications 1 à 24, comprenant l'insertion desdits bouchons d'oreilles (10, 12) dans ledit canal intra-auriculaire de l'utilisateur pour atténuer mécaniquement les ondes sonores arrivant au niveau de l'extrémité externe de chaque bouchon d'oreille d'au moins 10 dB en moyenne dans la gamme des fréquences audibles, l'établissement d'une communication électromagnétique sans fil entre ledit dispositif de protection de l'ouïe et ladite unité distante (36, 38, 40) via ladite antenne (24), et la fourniture de signaux acoustiques à l'utilisateur via ledit au moins un transducteur de sortie acoustique (32).

26. Utilisation selon la revendication 25, comprenant, en outre : la programmation et/ou la configuration d'une unité de traitement de signaux numériques (28) prévue dans l'un au moins desdits bouchons d'oreilles (10, 12) par des signaux en provenance de ladite unité distante (38) via ladite antenne (24).

27. Utilisation d'un dispositif de protection de l'ouïe selon l'une des revendications 1 à 24, comprenant : l'insertion desdits bouchons d'oreilles (10, 12) dans ledit canal intra-auriculaire de l'utilisateur pour atténuer mécaniquement les ondes sonores arrivant au niveau de l'extrémité externe de chaque bouchon d'oreille d'au moins 10 dB en moyenne dans la gamme des fréquences audibles, l'établissement d'une communication électromagnétique sans fil entre ledit dispositif de protection de l'ouïe et ladite unité distante (40) via ladite antenne (24), et la transmission de signaux audio produits par ledit microphone (30) à ladite unité distante, et la fourniture desdits signaux audio à l'oreille d'un autre utilisateur via au moins un transducteur de sortie acoustique.
